# EUROPEAN PATENT SPECIFICATION

(11) **EP 2 720 723 B1**
(45) Date of publication and mention of the grant of the patent: **11.04.2018**
(21) Application number: 11727955.4
(22) Date of filing: 15.06.2011
(51) Int. Cl.: A61K 9/19, A61K 9/00, A61K 47/26, A61K 47/69

(54) **STABILIZED VORICONAZOLE COMPOSITION**
STABILISIERTE VORICONAZOLZUSAMMENSETZUNG
COMPOSITION DE VORICONAZOLE STABILISÉE

(43) Date of publication of application: 23.04.2014
(73) Proprietor: Synthon BV, 6545 CM Nijmegen (NL)
(72) Inventor: ZALUDEK, Borek, 67817 Blansko (CZ); ZATLOUKALOVA, Libuse, 67817 Blansko (CZ)
(74) Representative: Mendivil Gil, Maria Dolores
(86) International application number: PCT/EP2011/059945
(87) International publication number: WO 2012/171561

(56) References cited:
- EP-A1- 2 018 866
- WO-A1-2011/020605
- CN-A- 1 788 725
- Herman B D ET AL: "The Effect of Bulking Agent on the Solid-State Stability of Freeze-Dried Methylprednisolone Sodium Succinate", Pharmaceutical Research, vol. 11, no. 10, 1 January 1994 (1994-01-01), pages 1467-1473, XP055200606, US ISSN: 0724-8741

## Description

### SUMMARY OF THE INVENTION

The present invention relates to a process for improving the stability of voriconazole in pharmaceutical compositions comprising voriconazole and a beta-cyclodextrin.

### BACKGROUND OF THE INVENTION

Voriconazole, chemically (2R,3S)-2-(2,4-difluorophenyl)-3-(5-fluoropyrimidin-4-yl)-1-(1H-1,2,4-triazol-1-yl)-butan-2-ol of the following formula 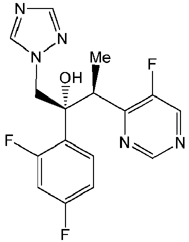 is a pharmaceutically active compound, which has been developed as an antifungal agent for the manufacture of a medicament for treatment of serious systemic fungal infections. The primary mode of action of voriconazole is the inhibition of fungal cytochrome P-450-mediated 14 alpha-lanosterol demethylation, an essential step in fungal ergosterol biosynthesis.

Voriconazole was generically disclosed within teaching of EP 357241 (US 5,116,844) and specifically disclosed in EP 440372 and in Bioorganic & Medicinal Chemistry Letters, 1996, 6, 2031. The compound may form acid addition salts (see, e.g. WO 97/06160, CN 1847243), however it is marketed as a free base. Two polymorphic forms of the base were disclosed in WO 2006/065726.

Voriconazole is poorly soluble in water (0.61 mg/ml at pH 7, 0.2 mg/ml at pH 3).

The marketed compositions sold e.g., under the brand name VFEND by Pfizer, comprise compositions both for oral and for intravenous administration. The composition developed for intravenous administration is a freeze-dried powder for infusion containing 200 mg of voriconazole in the form of a complex with 3200 mg of sulfobutyl ether β-cyclodextrin sodium. After reconstitution with water, it provides a parenteral solution of voriconazole with a concentration of 10 mg/ml.

Inclusion complexes of chemical compounds with cyclodextrins are well known in the art. Among their properties, the ability to improve aqueous solubility of the parent compound is well documented and used in practical applications. Thus, it is not surprising that the problem of inherently poor aqueous solubility of voriconazole was solved in medical preparations by using a specific kind of a cyclodextrin, which acts as a solubilizer.

Apart of low aqueous solubility, voriconazole also suffers from the problem of low stability. It breaks down by a retro-aldol reaction to compounds A and C (as labeled in Ph.Eur.) as shown in following scheme:

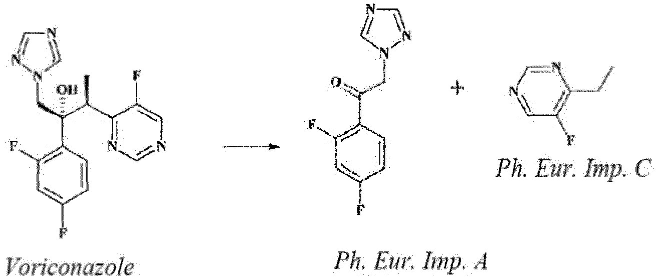

Subsequently, inactive enantiomers can be formed by recombination of the retro-aldol products A and C. This instability is pronounced not only in an aqueous media but also in solid state.

The sulfobutyl ether β-cyclodextrin, which is a specifically modified natural beta cyclodextrin, used in the above marketed medicinal product serves for the purpose of solubilization of voriconazole in water and also for its stabilization. Freeze-dried powders for infusion based on a complex of a drug with sulfobutyl ether β-cyclodextrin sodium were first disclosed in WO 91/11172 and divisional WO 94/02518. The marketed powder for infusion comprising the combination of voriconazole with sulfobutyl ether β-cyclodextrin sodium has been specifically disclosed in WO 98/58677.

While sulfobutyl ether β-cyclodextrin sodium was proven a useful agent for the solubilization and stabilization of voriconazole in aqueous solutions, it is relatively expensive. Thus, it would be beneficial to provide stable and soluble injectable voriconazole compositions employing cheaper cyclodextrin excipients. Not all cyclodextrin compounds may be used for this purpose as some of them, typically natural cyclodextrins, exhibit certain toxicity, particularly nephrotoxicity, which limits their use in parenteral preparations.

A cyclodextrin compound of the first choice is 2-hydroxypropyl beta-cyclodextrin. Apart of being cheaper, it is also less toxic than sulfobutyl ether β-cyclodextrin sodium and it has been approved for parenteral compositions. This cyclodextrin may form a soluble inclusion complex with voriconazole as shown in, e.g., EP 2018866, WO2011/020605 and/or CN 1788725. As found by the present inventors, such binary complex shows however instability not only in aqueous solutions but even in solid state, e,g, after lyophilization. No successful solution providing for a sufficiently stable composition of voriconazole with 2-hydroxypropyl-beta-cyclodextrin was, however, disclosed in the prior art.

### BRIEF DESCRIPTION OF THE PRESENT INVENTION

The present invention is based on the discovery of stabilizing effect of a certain amount of lactose added to the composition comprising an inclusion complex of voriconazole and hydroxypropyl-beta-cyclodextrin. Accordingly, the present invention provides a soluble composition comprising voriconazole with improved stability, said composition being useful in intravenous administration of voriconazole to a patient in need thereof.

According to a first aspect, the invention provides a process of stabilization of a composition comprising a mixture of voriconazole and hydroxypropyl-beta-cyclodextrin, such process comprising the steps of
- providing an aqueous solution comprising voriconazole and hydroxypropyl-beta-cyclodextrin,
- adding 4 to 10 parts by weight of lactose in respect to voriconazole, calculated as lactose monohydrate to said aqueous solution and,
- freeze drying the obtained stabilized composition.

According to a second aspect, the invention provides a stabilized composition for intravenous administration of voriconazole comprising one part by weight of voriconazole, 10 to 30 parts by weight of 2-hydroxypropyl-beta-cyclodextrin and at 4 to 10 parts by weight, of lactose, calculated as lactose monohydrate. In an advantageous aspect, the composition may be combined with an aqueous diluent providing a stabilized voriconazole solution comprising voriconazole in a concentration of 10 mg/ml, hydroxypropyl-beta cyclodextrin in a concentration of 100-300 mg/ml and lactose in a concentration of between 40 and 100 mg/ml (calculated as lactose monohydrate). Preferably, the 2-hydroxypropyl-beta-cyclodextrin is a high substituted 2-hydroxypropyl-beta-cyclodextrin, with the molar substitution value between 0.8 and 1.

The stabilized composition is intended for use as a medicament for treatment of various diseases, which are treatable by voriconazole, e.g. fungal infections.

### DETAILED DESCRIPTION OF THE INVENTION

The present invention relates to a stabilized composition comprising the pharmaceutically effective compound voriconazole, wherein such composition is intended for intravenous administration of an aqueous solution of voriconazole to a human patient having the need thereof. In particular, the composition is in a form of a freeze-dried powder. The powder may be dissolved in water and then diluted in a suitable liquid isotonic to human blood (typically 0.9% of aqueous NaCl or 5% of glucose) to provide a solution, which is administered to human patient by an intravenous infusion. (For more details see, e.g., L.A. Trissel, Handbook of injectable drugs, 15th. Edition 2009, p.1595-1596)

Voriconazole, as used therein, represents a free base of (2R,3S)-2-(2,4-difluorophenyl)-3-(5-fluoropyrimidin-4-yl)-1-(1H-1,2,4-triazol-1-yl)-butan-2-ol. Any solid state form, incl. hydrates and solvates, is equally useful for the purpose of the present invention.

As discussed above, inherently poor aqueous solubility of voriconazole may be significantly improved by combining voriconazole with a cyclodextrin. Cyclodextrins are a family of compounds made up of sugar molecules bound together in a ring. Cyclodextrins are composed of 5 or more α-D-glucopyranoside units linked 1->4. Cyclodextrins are produced from starch by means of enzymatic conversion. Three naturally occurred cyclodextrins are known, whereby the so-called beta-cyclodextrin has seven sugar molecules in the ring. As the natural beta-cyclodextrin exhibits relatively low aqueous solubility, various semi synthetic derivatives with enhanced aqueous solubility have been developed.

Cyclodextrins may form stable complexes with various chemicals, in which the molecule of the chemical is encapsulated inside of the cyclodextrin ring and forms a so called inclusion complex. Thereby, original properties of the chemical vis-a-vis the cyclodextrin-complexed chemical, particularly the aqueous solubility, may be modified. (For more details, see ,e.g., Arthur H. Kibbe (Ed.), Handbook of Pharmaceutical Excipients, Third Edition 2000, p.165-168).

At present, the stable and soluble voriconazole composition for intravenous administration, which is on the market, is a composition comprising an inclusion complex of 1 weight part of voriconazole and 16 weight parts of sulfobutyl ether β-cyclodextrin sodium (SB-β-CD). This semisynthetic cyclodextrin is relatively expensive; it has been chosen for the marketed composition probably because of an advantageous combination of solubilization and stabilization effects.

Compositions comprising an inclusion complex of voriconazole with hydroxypropyl-beta-cyclodextrin (HP-β-CD), which is a cheap semisynthetic derivative of natural beta-cyclodextrin with sufficient solubilization properties, are also known in the art. However, as will be shown in more detail below, stability studies showed that the stability, particularly the hydrolytic stability, of inclusion complexes with voriconazole is far lower than those comprising the SB-β-CD. Ways of improving their stability have not been disclosed in the literature. Thus, various possibilities of improving the stability of compositions comprising voriconazole and HP-β-CD were studied by the present inventors, resulting to a discovery of stabilizing effect of lactose.

Lactose is a known pharmaceutical excipient used in injectable compositions. However, to our knowledge, it has not been used as a stabilizer therein.

With a certain surprise, it was now found out by the present inventors that a certain amount of lactose, the "stabilizing amount" thereof, improves the stability of voriconazole with the inclusion complex with HP-β-CD.

Thus, in general, the present invention relates to a novel use of lactose. In particular, the present invention provides the use of lactose as an agent for stabilization of a composition, particularly a freeze-dried composition, comprising a mixture of voriconazole and hydroxypropyl-beta-cyclodextrin.

Hydroxypropyl-beta-cyclodextrin is a partially substituted poly(2-hydroxpropyl) ether of beta-cyclodextrin. It is commercially obtainable in various degrees of substitution with hydroxypropyl groups. Degree of substitution is normally expressed as a molar substitution (MS) value, which is number of hydroxypropyl groups per one glucose unit. Within the present invention, a highly substituted HP-β-CD (with MS 0.8 - 1) is preferred due to advantageous solubilization properties. Its aqueous solubility is quite high, exceeding 600 mg/ml.

Lactose is commercially obtainable both in a crystalline state and in an amorphous state; it is also obtainable either as an anhydrous product or as a monohydrate; next, it may form two stereoisomers, so called alpha- and beta-form. (For more details, see ,e.g., Arthur H. Kibbe (Ed.), Handbook of Pharmaceutical Excipients, Third Edition 2000, p.276-285). All these grades, incl. mixtures thereof, are equally suitable in the invention with the proviso that the used brand of lactose must be of a quality prescribed for use in parenteral applications. Because of its advantageous properties in handling, lactose monohydrate is preferred in the processes and compositions of the present invention. Accordingly, the weight parts of the lactose used in the stabilized composition are the weight parts of lactose monohydrate; if anhydrous lactose is used, the amounts have to be recalculated.

In particular, the stabilizing amount of lactose for the above purpose, calculated as lactose monohydrate, is from 4 to 10, weight parts in respect to voriconazole. The stabilizing effect of lactose was proven by subjecting the voriconazole-HP-β-CD freeze-dried compositions with and without lactose to stability studies according to ICH guidelines at 40°C and 75% relative humidity and at 50°C and dry heat. The results are summarized in the Table 1 below.

In particular, the relative amount of the HP-β-CD in the stabilized composition of the invention is 10 to 30 parts by weight in respect to voriconazole, advantageously between 13 to 24 parts by weight; such amount assures the necessary solubilization (at least 10 mg/ml) of voriconazole in water after reconstitution of the freeze-dried composition.

Accordingly, the present invention also provides for a novel process of stabilizing voriconazole in a complex with hydroxypropyl-beta-cyclodextrin, in particular within a freeze-dried composition comprising a mixture of voriconazole and HP-β-CD and intended for intravenous application of voriconazole. The process comprises the following steps:
a] providing an aqueous solution comprising voriconazole and hydroxypropyl-beta-cyclodextrin, preferably of a concentration of about 10 mg/ml of voriconazole,
b]adding the stabilizing amount of lactose to said aqueous solution and, optionally,
c] freeze drying the obtained stabilized composition.

Ad a] The calculated amount of 2-hydroxypropyl beta-cyclodextrin, which typically is 10 to 30 parts by weight, advantageously between 13 to 24 parts by weight, in respect to voriconazole, is dissolved in water, preferably at a temperature higher than 25°C, advantageously at a temperature between 30 to 40°C. Then the calculated amount of voriconazole, typically such for to obtain final concentration of the active substance of at least 10 mg /ml, is added and the mixture is stirred at the same temperature until the dissolution is complete. Dissolution process can take from 1 to 4 hours, preferably 2 to 3 hours.

Ad b] After the dissolution of voriconazole is finished, the effective stabilizing amount of lactose, which is from 4 to 10, weight parts (calculated as lactose monohydrate) in respect to voriconazole, is added and dissolved under stirring. Then the solution is cooled down under stirring to ambient temperature.

Ad c] If the solution is intended for making a product for intravenous application, the prepared solution is then sterilized ,preferably by filtration through a filter of pore size less than 0.2 microns. The solution is filled portion-wise into vials, preferably at aseptic conditions, and lyophilized. The dose unit within the vial typically comprises an amount comprising from 50 to 500 mg, typically about 200 mg, of voriconazole.

Optionally, the whole process can be done under protective atmosphere of nitrogen or other pharmaceutically acceptable inert gas.

The present invention also provides for a stabilized composition for intravenous administration of voriconazole. The composition comprises one part by weight of voriconazole, 10 to 30 parts by weight, advantageously between 13 to 24 parts by weight, of 2-hydroxypropyl-beta-cyclodextrin and from 4 to 10 parts by weight, of lactose. The highly substituted HP-β-CD (with molar substitution value of between 0.8 - 1) is preferred due to advantageous solubilization properties. Lactose monohydrate is the preferred type of lactose. The composition is typically a freeze-dried composition, prepared by a lyophilization of an aqueous solution comprising the above components. Typically, the composition is prepared by a process described above.

In an advantageous aspect, the composition may be combined with an aqueous diluent providing a stabilized voriconazole solution comprising voriconazole in a concentration of 10 mg/ml, hydroxypropyl-beta-cyclodextrin in a concentration of 100-300 mg/ml and lactose in a concentration between 40 and 100 mg/ml (calculated as lactose monohydrate). Advantageously, the stabilized voriconazole solution comprises from 50 to 500 mg, typically about 200 mg, of voriconazole.

The stabilized voriconazole solution may be further diluted with an isotonic solution for parenteral administration to an amount and/or concentration, which is prescribed for the treatment of the disease to be treated, to obtain a medicinal composition, which is administered to a patient in need of treatment by voriconazole.

The stabilized composition of the present invention and/or obtained by the process of the present invention can be used as an antifungal agent, typically within a pharmaceutical composition administered to a patient by an infusion. In a further aspect the present invention provides the use of a stabilized composition comprising voriconazole, hydroxypropyl-beta-cyclodextrin and the stabilizing amount of lactose according to the present invention or obtainable according to a process according to the present invention for treating a fungal infection, preferably wherein said composition is administered to a patient in need thereof by an intravenous infusion within a medicinal composition.

A suitable dose for the parenteral administration of voriconazole is between 0.1 to 25 mg/kg, such as 0.5 to 20 mg/kg, for example 1 to 10 mg/kg. The precise therapeutic dose of voriconazole will depend on the age and condition of the patient and the nature of the condition to be treated and will be at the ultimate discretion of the attendant physician.

The following examples illustrate the present invention and are not intended to limit the scope of the invention set forth in the claims.

### EXAMPLES

### Example 1 - Preparation of a stabilized freeze-dried composition comprising voriconazole, HP-β-CD, and lactose monohydrate in a weight ratio 1:13:6

### Procedure:

Water for injections was purged with nitrogen for 10 minutes.

Then 425 ml of that pre-treated water was put in a glass reaction vessel and heated to 35°C under stirring. The stirrer speed was adjusted to 350 rpm.

High-substituted hydroxypropyl-beta-cyclodextrine [Kleptose HP PF, molar substitution 0.9] 65.0 g (recalculated amount on moisture content) was added under stirring. It was dissolved within 10 minutes.

Then 5.0 g of voriconazole was added.

The temperature during the dissolution was kept at 35°C. The total time of dissolution was 2 hours.

30 g lactose monohydrate was added into the solution under stirring.

The temperature of the water bath was then set to 23°C and the solution was cooled down to 25°C within 1 hour.

The solution was transferred to volumetric flask and filled by water up to 500 ml.

The solution was filtered through filter with a pore size of 0.2 microns, filled in vials and lyophilized.

After lyophilization vials were closed under vacuum with rubber stoppers and crimped with aluminous cap.

The manipulation with the solution was made under inert atmosphere of nitrogen.

### Example 2 - Stability studies with voriconazole freeze-dried compositions

Various freeze-dried compositions with voriconazole (V), hydroxypropyl-beta-cyclodextrine (CD), with or without lactose monohydrate (L) in different weight ratios were prepared by the process of the Example 1:

| | | |
|---|---|---|
| Batch 1 | V:CD:L | 1:13:6 |
| Batch 2 | V:CD:L | 1 : 13 : 4 |
| Batch 3 | V:CD:L | 1:13:2 |
| Batch 4 | V:CD:L | 1 : 13 : 1 |
| Batch 5 | V:CD:L | 1 : 16 : 1 |

Comparative compositions cited by the literature were prepared by the above process of the Example 1:

| | | |
|---|---|---|
| Batch 6 | V: sulfobutylether beta-cyclodextrin | 1 : 16 (US 6,632,803) |
| Batch 7 | V : CD | 1 : 20 (CN1788725, example 1) |
| Batch 8 | V : CD : mannitol | 1:5:10 (CN1788725, example 2) |

| | | |
|---|---|---|
| Note: In this case partial dissolving of components was achieved only. The insoluble part was separated by filtration before filling in vials and lyophilization. | | |

| | | |
|---|---|---|
| Batch 9 | V: CD | 1 : 16 (EP 2018866) |

The vials comprising the freeze-dried powder were put into a thermostated chamber at 45°C/75% RH and 50°C/dry heat. The samples were analysed after a preselected time by HPLC. The peaks of impurities were evaluated by internal normalization and the overall impurities content was calculated.

**Table 1: Stability of compositions prepared by the procedure according to Example 1**

| Batch | 2 weeks 50°C Dry heat | 1 month, 40°C/75% RH |
|---|---|---|
| 1 | 0.29% | 0.21% |
| 2 | 0.39% | 0.27% |
| 3 | 0.64% | 0.39% |
| 4 | 0.93% | 0.54% |
| 5 | 0.99% | 0.57% |
| 6 | 0.46% | 0.26% |
| 7 | 1.36% | 0.78% |
| 8 | 6.43% | 4.85% |
| 9 | Not analyzed | 5.32% |

As apparent, lactose in a stabilizing amount (batches 1 and 2) results in a comparative stability with a composition comprising sulfobutylether beta-cyclodextrin (batch 6). Lactose in an amount less than the stabilizing amount (Batches 3,4,5) exhibit worse stability. Batches without the lactose (batches 7 and 9) and with mannitol instead of lactose (batch 8) exhibit the lowest stability.

## Claims

1. A process for stabilization of a composition comprising a mixture of voriconazole and hydroxypropyl-beta-cyclodextrin, such process comprising the steps of:
• Providing an aqueous solution comprising voriconazole and hydroxypropylbeta-cyclodextrin;
• Adding 4 to 10 parts by weight of lactose in respect to voriconazole, calculated as lactose monohydrate. to said aqueous solution;
• Freeze-drying the obtained stabilized composition.

2. A stabilized composition for intravenous administration of voriconazole comprising one part by weight of voriconazole, 10 to 30 parts by weight of 2-hydroxypropyl-betacyclodextrin and at least 4 to 10 by weight, of lactose, calculated as lactose monohydrate.

3. The composition according to claim 2 combined with an aqueous diluent providing a stabilized voriconazole solution comprising voriconazole in a concentration of 10 mg/ml, 2-hydroxypropyl-beta-cyclodextrin in a concentration of 100-300 mg/ml and lactose in a concentration of between 40 and 100 mg/ml calculated as lactose monohydrate.

4. The composition according to claim 3, wherein the 2-hydroxypropyl-betacyclodextrin is substituted 2-hydroxypropyl-beta-cyclodextrin with the molar substitution value between 0.8 and 1.

5. The composition according to claims 3-4 and/or prepared by the process of claim 1 for use in medicine, preferably for the treatment of fungal infections, which are treatable by voriconazole.

## Patentansprüche

1. Verfahren zur Stabilisierung einer Zusammensetzung, die ein Gemisch aus Voriconazol und Hydroxypropyl-beta-cyclodextrin umfasst, wobei das Verfahren die Schritte umfasst:
• Bereitstellen einer wässrigen Lösung, die Voriconazol und Hydroxypropyl-beta-cyclodextrin umfasst;
• Zugeben von 4 bis 10 Gewichtsteilen Lactose bezogen auf Voriconazol, berechnet als Lactose-Monohydrat, zu der wässrigen Lösung;
• Gefriertrocknen der erhaltenen stabilisierten Zusammensetzung.

2. Stabilisierte Zusammensetzung zur intravenösen Verabreichung von Voriconazol, die ein Gewichtsteil Voriconazol, 10 bis 30 Gewichtsteile 2-Hydroxypropyl-beta-cyclodextrin und mindestens 4 bis 10 Gewichtsteile Lactose, berechnet als Lactose-Monohydrat, umfasst.

3. Zusammensetzung nach Anspruch 2, kombiniert mit einem wässrigen Verdünnungsmittel, was eine stabilisierte Voriconazol-Lösung bereitstellt, die Voriconazol in einer Konzentration von 10 mg/ml, 2-Hydroxypropyl-beta-cyclodextrin in einer Konzentration von 100-300 mg/ml und Lactose in einer Konzentration von zwischen 40 und 100 mg/ml, berechnet als Lactose-Monohydrat, umfasst.

4. Zusammensetzung nach Anspruch 3, wobei das 2-Hydroxypropyl-beta-cyclodextrin substituiertes 2-Hydroxypropyl-beta-cyclodextrin mit dem molaren Substitutionswert zwischen 0,8 und 1 ist.

5. Zusammensetzung nach Ansprüchen 3-4 und/oder hergestellt durch das Verfahren gemäß Anspruch 1, zur Verwendung in der Medizin, bevorzugt für die Behandlung von Pilzinfektionen, die durch Voriconazol behandelbar sind.

## Revendications

1. Un procédé de stabilisation d'une composition comprenant un mélange de voriconazole et d'hydroxypropyl-bêta-cyclodextrine, ce procédé comprenant les étapes consistant à :
- fournir une solution aqueuse comprenant du voriconazole et de l'hydroxypropyl-bêta-cyclodextrine;
- ajouter à ladite solution aqueuse de 4 à 10 parties en poids de lactose par rapport au voriconazole, calculé par rapport au lactose monohydraté;
- lyophiliser la composition stabilisée obtenue.

2. Une composition stabilisée pour administration intraveineuse de voriconazole comprenant une partie en poids de voriconazole, 10 à 30 parties en poids de 2-hydroxypropyl-bêta-cyclodextrine et au moins 4 à 10 parties en poids de lactose, calculée par rapport au lactose monohydraté.

3. La composition selon la revendication 2, combinée avec un diluant aqueux fournissant une solution de voriconazole stabilisée comprenant du voriconazole à une concentration de 10 mg/ml, de la 2-hydroxypropyl-bêta cyclodextrine à une concentration de 100-300 mg/ml et du lactose à une concentration comprise entre 40 et 100 mg/ml, calculée par rapport au lactose monohydraté.

4. La composition selon la revendication 3, dans laquelle la 2-hydroxypropyl-bêta-cyclodextrine est une 2-hydroxypropyl-bêta-cyclodextrine substituée, dont la valeur de substitution molaire est comprise entre 0,8 et 1.

5. La composition selon les revendications 3-4 et/ou préparée par le procédé de la revendication 1, pour une utilisation en médecine, de préférence pour le traitement d'infections fongiques, qui peuvent être traitées par le voriconazole.
